# EUROPÄISCHE PATENTSCHRIFT

(11) **EP 1 736 306 B1**
(45) Veröffentlichungstag und Bekanntmachung des Hinweises auf die Patenterteilung: **31.03.2010**
(21) Anmeldenummer: 05013588.8
(22) Anmeldetag: 23.06.2005
(51) Int. Cl.: B32B 3/04, B32B 3/18, B32B 5/26

(54) **Verbundstoffbahn mit elastischen und unelastischen Bereichen**
Composite fabric with inelastic and elastic regions
Bande de tissu composite avec régions élastiques et non-élastiques

(43) Veröffentlichungstag der Anmeldung: 27.12.2006
(73) Patentinhaber: Nordenia Deutschland Gronau GmbH, 58599 Gronau (DE)
(72) Erfinder: Baldauf, Georg, 48366 Laer (DE); Leuders, Josef, 48599 Gronau-Epe (DE); Bader, Herbert Dr., 48565 Steinfurt-Borghorst (DE); Schönbeck, Marcus, 33775 Versmold (DE)
(74) Vertreter: Albrecht, Rainer Harald

(56) Entgegenhaltungen:
- EP-A- 1 331 090
- EP-A- 1 342 562
- EP-A- 1 342 563
- EP-A- 1 686 209

## Beschreibung

Die Erfindung betrifft eine Verbundstoffbahn mit elastischen und unelastischen Bereichen, die an beiden Oberflächen Vliesstoff und dazwischen abschnittsweise Folienstreifen einer elastischen Folie aufweist, wobei eine Oberfläche in Form einer flächigen Vliesstofflage vorliegt und wobei die Vliesstofflage zwischen den Folienstreifen mit Vliesstoff verbunden ist, der an der gegenüberliegenden Oberfläche der Verbundstoffbahn angeordnet ist, sowie ein Verfahren zur Herstellung der Verbundstoffbahn. Abschnitte einer solchen Verbundstoffbahn sind beispielsweise für die Fertigung von Hygieneprodukten geeignet, an denen sie als elastische Abschlussbereiche oder elastische Verschlussstreifen verwendet werden können.

Eine Verbundstoffbahn mit den eingangs beschriebenen Merkmalen, sowie ein Verfahren zu dessen Herstellung wird in DE 102 02 333 A1 beschrieben. Im Rahmen der bekannten Maßnahmen wird ein thermoplastisches Elastomer im schmelzflüssigen Zustand in Form von Streifen zwischen zwei Vliesstoffbahnen eingebracht, wobei die Vliesstoffbahnen zwischen den Streifen des thermoplastischen Elastomers flächig miteinander verklebt werden. Die Verbundstoffbahn ist im Bereich der Folienstreifen in Querrichtung elastisch und zwischen den Folienstreifen, wo die Vliesstoffbahnen flächig verklebt sind, unelastisch. Bei dem bekannten Verfahren zur Herstellung der Verbundstoffbahn muss die Extrusion des thermoplastischen Elastomers in einem Prozeß auf den Kaschiervorgang abgestimmt werden, wobei die Breite der elastischen Folienstreifen durch die Verwendung von speziellen Extrusionsdüsen vorgegeben ist. Das Material der Vliesstoffbahnen ist derart zu wählen, dass einerseits eine hohe Reißfestigkeit und andererseits eine hohe Elastizität erreicht werden können.

Der Erfindung liegt die Aufgabe zugrunde eine Verbundstoffbahn, sowie ein Verfahren zu dessen Herstellung anzugeben, die eine verbesserte Reißfestigkeit bei einer hohen Elastizität aufweist und kostengünstig sowie flexibel zu fertigen ist.

Ausgehend von einer Verbundstoffbahn mit den eingangs beschriebenen Merkmalen wird die Aufgabe erfindungsgemäß dadurch gelöst, dass jeder Folienstreifen mit jeweils einer Vliesstoffschicht zu einem Laminatstreifen verbunden ist, dass die Laminatstreifen auf ihrer Folienseite mit der flächigen Vliesstofflage verklebt sind und dass der Vliesstoff, der zwischen den Laminatstreifen mit der flächigen Vliesstofflage verbunden ist, in Form von Vliesstoffstreifen vorliegt, welche parallel zu den Laminatstreifen verlaufen und die Vliesstoffschicht der Laminatstreifen in Randbereichen der Laminatstreifen überlappen. Die erfindungsgemäße Verbundstoffbahn ist in den Bereichen der Laminatstreifen quer zu deren Längsrichtung elastisch und zwischen den Laminatstreifen unelastisch, wobei die elastischen Eigenschaften und die Reißfestigkeit der Verbundstoffbahn sowohl durch den Aufbau der Laminatstreifen als auch durch den Aufbau der Vliesstoffstreifen gezielt verändert werden können.

Vorzugsweise sind die Vliesstoffstreifen zwischen den Laminatstreifen vollflächig mit der flächigen Vliesstofflage verklebt. Die Erfindung umfasst jedoch auch andere Verbindungsarten wie beispielsweise eine Verbindung durch Ultraschallschweißen oder thermisches Schweißen.

Der Folienstreifen eines Laminatstreifens besteht vorzugsweise aus einer Monofolie eines thermoplastischen Elastomers, wobei im besonderen Folienstreifen aus einem Polymer der Gruppe der Styrol-Butadien-Styrol-Copolymere (SBS), Styrol-Isopren-Styrol-Block-Copolymere (SIS), Styrol-Ethenbuten-Styrol-Copolymere (SEBS), elastischen Polyetylen-Copolymere, elastischen Polypropylen-Copolymere, elastischen Polyurethan-Copolymere, elastischen Polyamid-Copolymere, oder eine Mischung dieser Polymere geeignet sind. Neben der Verwendung von Monofolien können auch coextrudierte Folien eingesetzt werden, wobei besonders coextrudierte Folien mit mehreren identischen Schichten geeignet sind. Besonders geeignet sind Folienstreifen mit einer Dicke zwischen 5 und 150 µm, vorzugsweise von mehr als 10 µm und weniger als 130 µm.

Die aus einer elastischen Folie und aufkaschiertern Vliesstoff bestehenden Laminatstreifen sind ein Vorprodukt, welches von der Rolle verarbeitet werden kann. Der Vliesstoff der Laminatstreifen ist mit der elastischen Folie verklebt, wobei vorzugsweise Schmelzklebstoffe zur Anwendung kommen. Besonders gute elastische Eigenschaften der Verbundstoffbahn können erreicht werden, wenn die beiden Schichten des Laminatstreifens durch gradlinige oder wellenförmige Klebstoffstreifen verbunden sind, die entlang der Richtung des Laminatstreifens verlaufen. In einer bevorzugten Ausführung der Erfindung ist der Vliesstoff der Laminatstreifen ein elastisches Spinnvlies, wobei jedoch auch kardierte Vliesstoffe oder unelastische Spinnvliesstoffe aus Polypropylen, Polyethylen, Polyamid oder Polyethylenterephthalat eingesetzt werden können. Im besonderen Maße geeignet sind elastische Spinnvliesstoffe auf der Basis von Kern-Mantel-Multifilamenten mit elastischen Kernpolymeren wie elastischen Polypropylen-Copolymeren oder Polyurethanen, wobei als Mantelpolymer beispielsweise Polypropylen oder Polyethylen eingesetzt wird. Neben Laminatstreifen, bei denen die Folienstreifen mit der Vliesstoffschicht verklebt sind, können im Rahmen der Erfindung auch Laminatstreifen eingesetzt werden, bei denen die Folienstreifen mit der Vliesstoffschicht ohne Klebstoff durch eine Extrusionskaschierung verbunden sind. Obwohl thermoplastische Elastomere typischerweise eine große Klebrigkeit aufweisen, können die verwendeten Laminate als Vorprodukt auf Rollen aufgerollt, gelagert, transportiert und verarbeitet werden, da durch die Vliesstoffschicht einerseits ein Verblocken des Laminates effektiv verhindert wird und andererseits die Zugfestigkeit des Laminates erhöht wird.

Die flächige Vliesstofflage weist typischerweise ein Flächengewicht zwischen 5 g/m² und 50 g/m², vorzugsweise zwischen 20 g/m² und 40 g/m² auf, wobei vorzugsweise ein kardierter Faservliesstoff eingesetzt wird. Neben kardierten Faservliesstoffen können beispielsweise auch Spinnvliesstoffe eingesetzt werden, die typischerweise ein Flächengewicht zwischen 10 g/m² und 30 g/m², aufweisen. Die flächige Vliesstofflage besteht beispielsweise aus Fasern aus Polyethylenterephthalat, Polyamid, Polyethylen, vorzugsweise aus Polypropylen.

Die Laminatstreifen sind durch einen Klebstoff, vorzugsweise einen Schmelzklebstoff, an ihrer Folienseite mit der flächigen Vliesstofflage verbunden, wobei der Klebstoff vorzugsweise in Form von gradlinigen oder wellenförmigen Klebstoffstreifen vorliegt, die entlang der Richtung der Laminatstreifen verlaufen. Zwischen den Laminatstreifen wird die Vliesstofflage mit Vliesstoffstreifen verbunden. In einer bevorzugten Ausführung der Erfindung wird der Klebstoff mit einem Flächengewicht zwischen 2 g/m² und 20 g/m², vorzugsweise zwischen 5 g/m² und 10 g/m², vor der Verbindung der Vliesstofflage mit den Laminatstreifen und den Vliesstoffstreifen auf die flächige Vliesstofflage aufgebracht. Vorteilhaft sind Vliesstoffstreifen aus einem leichtgewichtigen Spinnvliesstoff mit einem Flächengewicht zwischen 10 g/m² und 30 g/m², vorzugsweise von mehr als 10 g/m² und weniger als 20 g/m². Geeignet ist beispielsweise ein Spinnvliesstoff aus Polypropylen mit einer hohen Reißfestigkeit. Alternativ können auch kardierte Faservliesstoffe aus Polypropylen, Polyethylen, Polyamid oder Polyethylenterephthalat eingesetzt werden. Die Randbereiche, in denen die Vliesstoffstreifen die Vliesstoffschicht der Laminatstreifen überlappen, kann eine Breite von 2 bis 7 mm, vorzugsweise mehr als 4 und weniger als 5 mm, aufweisen. Die Vliesstoffstreifen sind zweckmäßigerweise fest, beispielsweise durch Klebstoff oder durch eine Verbindung durch Ultraschallschweißen, mit der Vliesstoffschicht der Laminatstreifen verbunden. Bei der Verwendung von Klebstoff sind in einer bevorzugten Ausführung der Erfindung die Vliesstoffstreifen und die Vliesstoffschicht der Laminatstreifen in den Randbereichen ausgehend von dem Rand des Vliesstoffstreifens auf einer Breite von maximal 5 mm, vorzugsweise auf einer Breite zwischen 1 und 4 mm, nicht miteinander verklebt. Bei dem alternativen Einsatz eines Ultraschallverfahrens erstreckt sich die Verbindung vorzugsweise bis zum Rand des Vliesstoffstreifens, wodurch die Reißfestigkeit der Verbindung erhöht ist.

Gegenstand der Erfindung ist auch ein Verfahren zur Herstellung der beschriebenen Verbundstoffbahn, wobei beabstandete, parallel verlaufende Laminatstreifen aus jeweils einem Folienstreifen einer elastischen Folie und einer aufkaschierten Vliesstoffschicht an ihrer Folienseite mit einer flächigen Vliesstofflage verklebt werden, wobei zwischen den Laminatstreifen Vliesstoffstreifen auf die flächige Vliesstofflage aufgebracht werden, und wobei die Vliesstoffstreifen die Vliesstoffschicht der Laminatstreifen in einem Randbereich der Laminatstreifen überlappen.

In einer bevorzugten Ausgestaltung des erfindungsgemäßen Verfahrens wird ein Laminat aus einer elastischen Folie, vorzugsweise einer elastischen extrudierten Monofolie aus einem thermoplastischen Elastomer, und einer Vliesstoffschicht von einer Rolle abgezogen und in Laminatstreifen geschnitten. Folie und Vlies des Laminats sind zweckmäßig durch streifenförmig aufgetragenen Klebstoff verbunden, wobei die Klebstoffstreifen sich in Längsrichtung der Laminatstreifen erstrecken. Eine vorteilhafte Ausgestaltung des erfindungsgemäßen Verfahrens sieht vor, dass Klebstoff, beispielsweise ein Schmelzklebstoff, in Bereichen, in denen die Laminatstreifen aufkaschiert werden, streifenförmig auf die Vliestofflage aufgebracht wird. In einer bevorzugten Ausführung der Erfindung wird der Klebstoff auch vollflächig in den Bereichen zwischen den Laminatstreifen aufgetragen. Die Erfindung ist jedoch nicht auf eine Verbindung durch Klebstoff begrenzt. So kann die Verbindung auch in einer Ultraschallschweißeinheit erzeugt werden.

Die Vliesstoffstreifen werden vorzugsweise ihrerseits auch mit einem Klebstoff, beispielsweise einem Schmelzklebstoff, versehen, der beispielsweise in Form von zwei Streifen auf die Seite der Vliesstoffstreifen aufgebracht wird, die auf die Vliesstofflage und die Randbereiche der Laminatstreifen kaschiert wird. Die Laminatstreifen und die Vliesstoffstreifen werden derartig auf die Vliesstofflage aufgebracht, dass diese sich in einem Randbereich mit einer Breite zwischen 2 und 7 mm, vorzugsweise mehr als 4 mm und weniger als 5 mm überlappen. Wird der Klebstoff mit einem Abstand von maximal 5 mm, vorzugsweise zwischen 1 und 4 mm zum Rand der Vliesstoffstreifen auf die Vliesstoffstreifen aufgebracht, kann verhindert werden, dass bei dem Kaschierprozess Klebstoff an der Oberfläche der Verbundstoffbahn austritt. Werden die Vliesstoffstreifen alternativ durch eine Ultraschallschweißstation mit der Vliesstoffschicht verbunden, so erstreckt sich die Verbindung vorzugsweise bis zu dem Rand der Vliesstoffstreifen. Wenn die Vliesstoffstreifen ohne die Verwendung von Klebstoff durch eine Ultraschallschweißstation mit den Laminatstreifen und der flächigen Vliesstofflage verbunden werde, kommt es bei einem Abriss oder bei einem Verlust der Vliesstoffstreifen während des Fertigungsprozesses nicht zu einer Verschmutzung der nachfolgenden Walzen.

Um die elastischen Eigenschaften der Verbundstoffbahn zu verbessern oder an bestimmte Vorgaben anzupassen, kann die Verbundstoffbahn nach der Laminierung quer zur Ausrichtung der Laminatstreifen, vorzugsweise in einer Reckwalzenanordnung, überdehnt werden.

Im Folgenden wird die Erfindung anhand einer lediglich ein Ausführungsbeispiel darstellenden Zeichnung ausführlicher erläutert. Es zeigen:
- **Fig. 1**: die Bestandteile einer Verbundstoffbahn in einer Schnittdarstellung,
- **Fig. 2**: eine Schnittdarstellung einer Verbundstoffbahn,
- **Fig. 3**: Schema des erfindungsgemäßen Verfahrens,
- **Fig. 4**: die Bestandteile der Verbundstoffbahnen in einer Schnittdarstellung bei einer alternativen Ausführung.

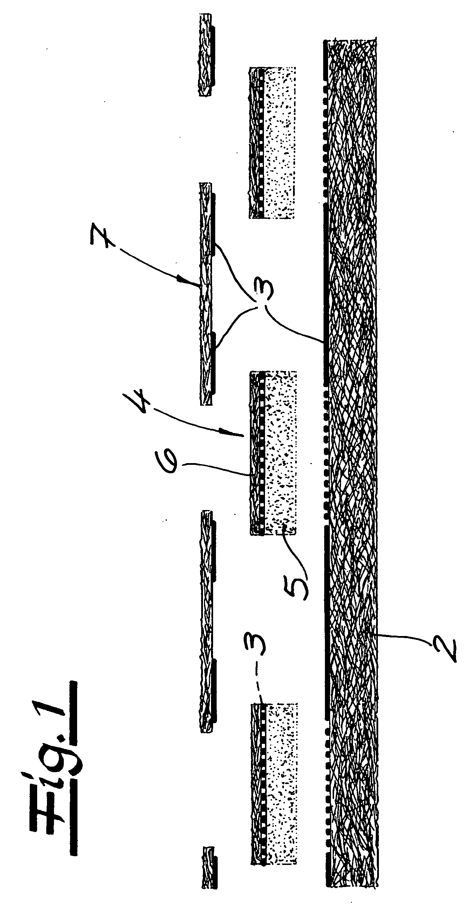

Die Bestandteile der Verbundstoffbahn 1 sind in Fig. 1 dargestellt. Auf eine flächige Vliesstofflage 2 ist ein Klebstoff 3 aufgetragen, der in den Bereichen, in denen Laminatstreifen 4 aufkaschiert werden, in Form von gradlinigen oder wellenförmigen Klebstoffstreifen aufgetragen ist, die entlang der Richtung der Laminatstreifen 4 verlaufen. Zwischen den Bereichen, in denen die Laminatstreifen 4 aufkaschiert werden, ist der Klebstoff 3 vollflächig auf die Vliesstofflage 2 aufgebracht. Jeder der Laminatstreifen 4 besteht aus einem Folienstreifen 5 aus einer Monofolie eines thermoplastischen Elastomers und einer Vliesstoffschicht 6 aus einem elastischen Spinnvliesstoff auf Basis von Kern-Mantel-Multifilamenten. Die Lagen des Laminatstreifens 4 sind durch Klebstoffstreifen, die entlang der Längsrichtung der Laminatstreifen 4 verlaufen, verbunden. Die Folienstreifen 5 aus einer Monofolie eines thermoplastischen Elastomers bestehen aus einem Polymer der Gruppe der Styrol-Butadien-Styrol-Copolymere (SBS), Styrol-Isopren-Styrol-Block-Copolymere (SIS), Styrol-Ethenbuten-Styrol-Copolymere (SEBS), elastischen Polyetylen-Copolymere, elastischen Polypropylen-Copolymere, elastischen Polyurethan-Copolymere, elastischen Polyamid-Copolymere, oder einer Mischung dieser Polymere. Auf die Vliesstoffstreifen 7 aus einem Spinnvliesstoff ist auf der Unterseite ein Klebstoff 3, vorzugsweise ein Schmelzklebstoff, in Form von zwei Streifen aufgetragen, wobei diese Streifen vom Rand der Vliesstoffstreifen 7 jeweils einen Abstand von maximal 5 mm, vorzugsweise von 1 bis 4 mm, aufweisen.

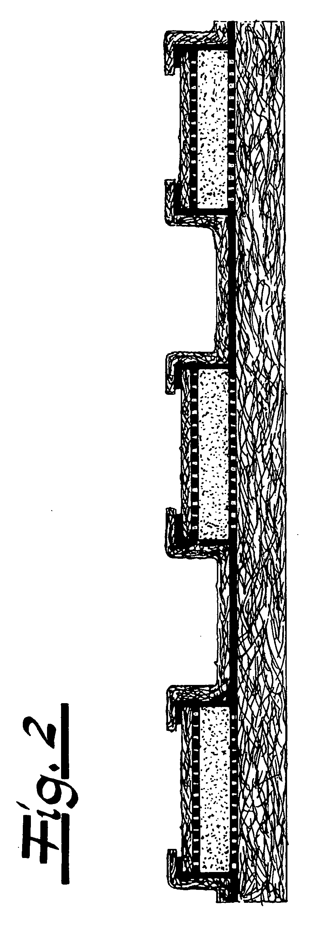

In Fig. 2 ist eine Verbundstoffbahn 1 dargestellt, die aus den in Fig. 1 dargestellten Bestandteilen gefertigt ist. Die Randbereiche, in dem die Vliesstoffstreifen 7 die Vliesstoffschicht 6 der Laminatstreifen 4 überlappen, weisen eine Breite von beispielsweise 2 bis 7 mm auf. Ausgehend von den Rändern der Vliesstoffstreifen 7 sind die Vliesstoffstreifen 7 in den Randbereichen auf einer Breite von maximal 5 mm nicht mit der darunter liegenden Vliesstoffschicht 6 der Laminatstreifen 4 verklebt. In den Bereichen der Laminatstreifen 4 ist die Verbundstoffbahn 1 quer zur Längsrichtung der Laminatstreifen 4 elastisch. Zwischen den Laminatstreifen 4, wo die Vliesstoffstreifen 7 vollflächig mit der flächigen Vliesstofflage 2 verklebt sind, ist die Verbundstoffbahn 1 unelastisch.

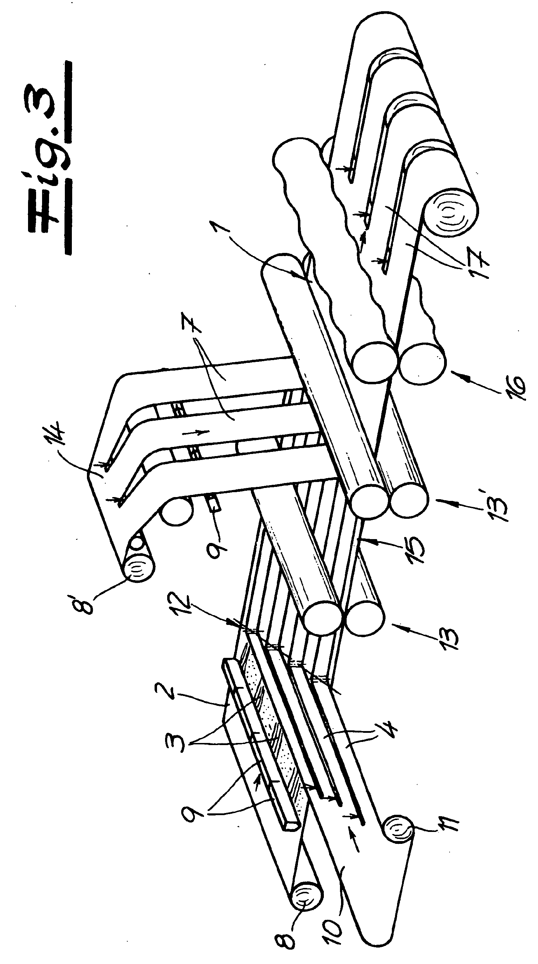

Das erfindungsgemäße Verfahren zur Herstellung einer Verbundstoffbahn 1 ist in Fig. 3 schematisch dargestellt. Die Vliesstofflage 2 wird von einer Vliesstoffrolle 8 abgerollt und mit Klebstoff 3 versehen, wobei der Klebstoff 3 in den Bereichen, in denen die Laminatstreifen 4 aufkaschiert werden, streifenförmig und in den Bereichen zwischen den Laminatstreifen 4 vollflächig von Klebstoffdüsen 9 aufgebracht. Ein Laminat 10 aus einer elastischen extrudierten Monofolie aus einem thermoplastischen Elastomer und einer Vliesstoffschicht 6 wird von einer Laminatrolle 11 abgezogen und in Laminatstreifen 4 geschnitten. Die Laminatstreifen 4 werden von einer Umlenkeinrichtung 12 beabstandet und parallel zueinander der mit Schmelzklebstoff versehenen Vliesstofflage 2 zugeführt und in einem Kaschierwerk 13 mit dieser verbunden. Vliesstoff 14 wird von einer Vliesstoffrolle 8' abgezogen und in Vliesstoffstreifen 7 geschnitten, die jeweils mit Hilfe von Klebstoffdüsen 9 mit zwei Streifen von Heißkleber versehen werden, wobei der Klebstoff 3 mit einem Abstand von maximal 5 mm zum Rand der Vliesstoffstreifen 7 aufgebracht wird. Die Vliesstoffstreifen 7 werden der Materialbahn 15 zugeführt und derart auf die Vliesstofflage 2 aufgebracht, dass sie die Vliesstoffschicht 6 der Laminatstreifen 4 in den Randbereichen der Laminatstreifen mit einer Breite zwischen 2 und 7 mm überlappen. Nach dem Durchlauf eines weiteren Kaschierwerkes 13' wird die Verbundstoffbahn 1 einer Reckwalzenanordnung 16 zugeführt, in der sie quer zu ihrer Laufrichtung überdehnt wird. Die Verbundstoffbahn 1 wird anschließend in ihrer Längsrichtung in Verbundstoffstreifen 17 geschnitten und aufgerollt. Abschnitte dieser Verbundstoffstreifen 17 können beispielsweise für den elastischen Abschluss oder Verschlussteile von Hygieneprodukten, wie beispielsweise Einwegwindeln, verwendet werden.

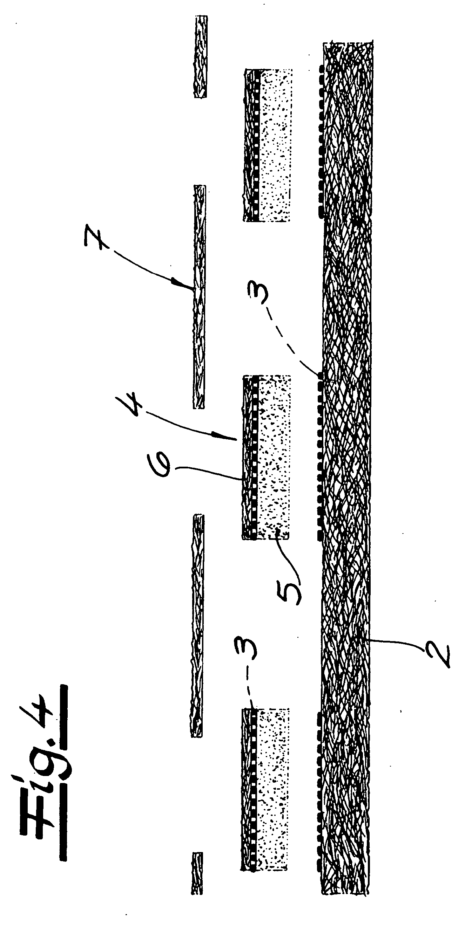

Die Fig. 4 zeigt die Bestandteile der Verbundstoffbahn 1, wobei die Vliesstoffstreifen 7 in einer alternativen Ausführung der Erfindung durch Ultraschallschweißen mit der Vliesstoffschicht 6 der Laminatstreifen 4 und mit der flächigen Vliesstofflage 2 verbunden werden. Lediglich im Bereich der Laminatstreifen 4 ist Klebstoff 3 auf die flächige Vliesstofflage 2 aufgetragen. Dadurch, dass in dem Bereich zwischen den Laminatstreifen 4 kein Klebstoff 3 vorliegt, wird die Gefahr einer Verschmutzung der verschiedenen Maschinenteile weitgehend ausgeschlossen. Bei dem Verfahren zur Herstellung der Verbundstoffbahn 1 werden die Vliesstoffstreifen 7 der Materialbahn 15 zugeführt und in einer Ultraschallschweißstation mit der Vliesstoffschicht 6 der Laminatstreifen 4 und der flächigen Vliesstofflage 2 verbunden.

## Patentansprüche

1. Verbundstoffbahn (1) mit elastischen und unelastischen Bereichen, die an beiden Oberflächen Vliesstoff und dazwischen abschnittsweise Folienstreifen (5) einer elastischen Folie aufweist, wobei eine Oberfläche in Form einer flächigen Vliesstofflage (2) vorliegt und wobei die Vliesstofflage (2) zwischen den Folienstreifen (5) mit Vliesstoff verbunden ist, der an der gegenüberliegenden Oberfläche der Verbundstoffbahn (1) angeordnet ist, **dadurch gekennzeichnet, dass** jeder Folienstreifen (5) mit jeweils einer Vliesstoffschicht (6) zu einem Laminatstreifen (4) verbunden ist, dass die Laminatstreifen (4) auf ihrer Folienseite mit der flächigen Vliesstofflage (2) verklebt sind und dass der Vliesstoff, der zwischen den Laminatstreifen (4) mit der flächigen Vliesstofflage (2) verbunden ist, in Form von Vliesstoffstreifen (7) vorliegt, welche parallel zu den Laminatstreifen (4) verlaufen und die Vliesstoffschicht (6) der Laminatstreifen (4) in Randbereichen der Laminatstreifen (4) überlappen.

2. Verbundstoffbahn (1) nach Anspruch 1, **dadurch gekennzeichnet, dass** die Vliesstoffstreifen (7) zwischen den Laminatstreifen (4) mit der flächigen Vliesstofflage (2) durch Ultraschallschweißen verbunden oder vollflächig verklebt sind.

3. Verbundstoffbahn (1) nach Anspruch 1 oder 2, **dadurch gekennzeichnet, dass** die Folienstreifen (5) aus einer Monofolie eines thermoplastischen Elastomers bestehen.

4. Verbundstoffbahn (1) nach Anspruch 3, **dadurch gekennzeichnet, dass** die Folienstreifen (5) aus einem Polymer der Gruppe der Styrol-Butadien-StyrolCopolymere (SBS), Styrol-Isopren-Styrol-Block-Copolymere (SIS), Styrol-Ethenbuten-Styrol-Copolymere (SEBS), elastischen Polyetylen-Copolymere, elastischen Polypropylen-Copolymere, elastischen Polyurethan-Copolymere, elastischen Polyamid-Copolymere, oder einer Mischung dieser Polymere besteht.

5. Verbundstoffbahn (1) nach einem der Ansprüche 1 bis 4, **dadurch gekennzeichnet, dass** die Folienstreifen (5) eine Dicke zwischen 5 und 150 µm, vorzugsweise von mehr als 10 µm und weniger als 70 µm, aufweisen.

6. Verbundstoffbahn (1) nach einem der Ansprüche 1 bis 5, **dadurch gekennzeichnet, dass** die flächige Vliesstofflage (2) ein Flächengewicht zwischen 5 g/m² und 50 g/m², vorzugsweise zwischen 20 g/m² und 40 g/m², aufweist.

7. Verbundstoffbahn (1) nach einem der Ansprüche 1 bis 6, **dadurch gekennzeichnet, dass** die Laminatstreifen (4) mit geradlinigen oder wellenförmigen Klebstoffstreifen, die entlang der Richtung der Laminatstreifen (4) verlaufen, mit der flächigen Vliesstofflage (2) verklebt sind.

8. Verbundstoffbahn (1) nach einem der Ansprüche 1 bis 7, **dadurch gekennzeichnet, dass** die Folienstreifen (5) und die Vliesstoffschicht (6) der Laminatstreifen (4) nicht vollflächig, vorzugsweise streifenförmig, verbunden sind.

9. Verbundstoffbahn (1) nach einem der Ansprüche 1 bis 8, **dadurch gekennzeichnet, dass** die Vliesstoffschicht (6) der Laminatstreifen (4) ein elastischer Spinnvliesstoff auf Basis von Kern-Mantel-Multifilamenten ist.

10. Verbundstoffbahn (1) nach einem der Ansprüche 1 bis 9, **dadurch gekennzeichnet, dass** die Randbereiche, in denen die Vliesstoffstreifen (7) die Vliesstoffschicht (6) der Laminatstreifen (4) überlappen, eine Breite von 2 bis 7 mm, vorzugsweise mehr als 4 und weniger als 5 mm, aufweisen.

11. Verbundstoffbahn (1) nach einem der Ansprüche 1 bis 10, **dadurch gekennzeichnet, dass** die Vliesstoffstreifen (7) in den Randbereichen der Laminatstreifen (4) mit der Vliesstoffschicht (6) verklebt sind.

12. Verbundstoffbahn (1) nach Anspruch 11, **dadurch gekennzeichnet, dass** die Vliesstoffstreifen (7) in den Randbereichen, in dem die Vliesstoffstreifen (7) die Vliesstoffschicht (6) der Laminatstreifen (4) überlappen, vom Rand des Vliesstoffstreifens (7) ausgehend auf einer Breite von maximal 5 mm, vorzugsweise 1 bis 4 mm, nicht mit der darunter liegenden Vliesstoffschicht (6) des Laminatstreifens (4) verklebt sind.

13. Verbundstoffbahn (1) nach einem der Ansprüche 1 bis 10, **dadurch gekennzeichnet, dass** die Vliesstoffstreifen (7) in dem Randbereichen der Laminatstreifen (4) durch Ultraschallschweißen mit der Vliesstoffschicht (6) verbunden sind.

14. Verbundstoffbahn (1) nach einem der Ansprüche 1 bis 10, **dadurch gekennzeichnet, dass** die Vliesstoffstreifen (7) aus einem Spinnvliesstoff mit einem Flächengewicht zwischen 10 und 30 g/m², vorzugsweise von mehr als 10 g/m² und weniger als 20 g/m², bestehen.

15. Verfahren zur Herstellung einer Verbundstoffbahn (1) nach einem der Ansprüche 1 bis 14,
wobei beabstandete, parallel verlaufende Laminatstreifen (4) aus jeweils einem Folienstreifen (5) einer elastischen Folie und einer aufkaschierten Vliesstoffschicht (6) an ihrer Folienseite mit einer flächigen Vliesstofflage (2) verklebt werden,
wobei zwischen den Laminatstreifen (4) Vliesstoffstreifen (7) auf die flächige Vliesstofflage (2) aufgebracht werden, und
wobei die Vliesstoffstreifen (7) die Vliesstoffschicht (6) der Laminatstreifen (4) in Randbereichen der Laminatstreifen (4) überlappen.

16. Verfahren nach Anspruch 15, **dadurch gekennzeichnet, dass** ein Laminat (10) aus einer elastischen extrudierten Monofolie aus einem thermoplastischen Elastomer und einer Vliesstoffschicht (6) von einer Rolle (11) abgezogen und in Laminatstreifen (4) geschnitten wird.

17. Verfahren nach Anspruch 15 oder 16, **dadurch gekennzeichnet, dass** der Folienstreifen (5) und die Vliesstoffschicht (6) jedes Laminatstreifens (4) durch Klebstoffstreifen verbunden sind, die in Längsrichtung des Laminatstreifens (4) verlaufen.

18. Verfahren nach einem der Ansprüche 15 bis 17, **dadurch gekennzeichnet, dass** Klebstoff (3) in dem Bereich, in dem Laminatstreifen (4) aufkaschiert werden, in Form von Klebstoffstreifen, die parallel zu den Laminatstreifen (4) verlaufen, und zwischen den Laminatstreifen (4) vollflächig auf die flächige Vliesstofflage (2) aufgebracht wird.

19. Verfahren nach einem der Ansprüche 15 bis 18, **dadurch gekennzeichnet, dass** Klebstoff (3) in dem Bereich zwischen den Laminatstreifen (4) vollflächig auf die flächige Vliesstofflage (2) aufgebracht wird.

20. Verfahren nach einem der Ansprüche 15 bis 19, **dadurch gekennzeichnet, dass** die Vliesstoffstreifen (7) derart auf die Vliesstofflage (2) aufgebracht werden, dass sie die Vliesstoffschicht (6) der Laminatstreifen (4) in den Randbereichen der Laminatstreifen (4) mit einer Breite zwischen 2 und 7 mm, vorzugsweise mehr als 4 und weniger als 5 mm, überlappen.

21. Verfahren nach einem der Ansprüche 15 bis 20, **dadurch gekennzeichnet, dass** Klebstoff (3) in Form von jeweils zwei Klebstoffstreifen auf die Vliesstoffstreifen (7) aufgebracht wird.

22. Verfahren nach einem der Ansprüche 15 bis 21, **dadurch gekennzeichnet, dass** Klebstoff (3) mit einem Abstand von maximal 5 mm, vorzugsweise 1 bis 4 mm, zum Rand der Vliesstoffstreifen (7) auf die Vliesstoffstreifen (7) aufgebracht wird.

23. Verfahren nach einem der Ansprüche 15 bis 20, **dadurch gekennzeichnet, dass** die Vliesstoffstreifen (7) zumindest in den Randbereichen der Laminatstreifen (4) durch Ultraschallschweißen mit der Vliesstoffschicht (6) verbunden werden.

24. Verfahren nach einem der Ansprüche 15 bis 23, **dadurch gekennzeichnet, dass** die Verbundstoffbahn (1) quer zur Ausrichtung der Laminatstreifen (4), vorzugsweise in einer Reckwalzenanordnung (16), überdehnt wird.

## Claims

1. A composite web (1) with elastic and inelastic regions, which on both surfaces has non-woven fabric and in between, by sections, film strips (5) of an elastic film, wherein a surface is present in the form of a planar non-woven fabric lay (2), and wherein the non-woven fabric lay (2) is joined between the film strips (5) with non-woven fabric, which is arranged on the opposite surface of the composite web (1), **characterised in that** each film strip (5) is joined in each case with a non-woven fabric layer (6) to form a laminated strip (4), **in that** the laminated strips (4) on their film side are bonded with the planar non-woven fabric lay (2), and **in that** the non-woven fabric, which is joined between the laminated strips (4) with the planar non-woven fabric lay (2), is present in the form of non-woven fabric strips (7), which run parallel to the laminated strips (4) and overlap the non-woven fabric layer (6) of the laminated strips (4) in edge regions of the laminated strips (4).

2. The composite web (1) according to Claim 1, **characterised in that** the non-woven fabric strips (7) are joined with the planar non-woven fabric lay (2) between the laminated strips (4) by means of ultrasound welding, or are bonded over the whole surface.

3. The composite web (1) according to Claim 1 or 2, **characterised in that** the film strips (5) consist of a mono-film of a thermoplastic elastomer.

4. The composite web (1) according to Claim 3, **characterised in that** the film strips (5) consist of a polymer of the group of styrene-butadiene-styrene copolymers (SBS), styrene-isoprene-styrene block copolymers (SIS), styrene-ethylene-butadiene-styrene copolymers (SEBS), elastic polyethylene copolymers, elastic polypropylene copolymers, elastic polyurethane copolymers, elastic polyamide copolymers, or a mixture of these polymers.

5. The composite web (1) according to one of the Claims 1 to 4, **characterised in that** the film strips (5) have a thickness of between 5 and 150 µm, preferably of more than 10 µm and less than 70 µm.

6. The composite web (1) according to one of the Claims 1 to 5, **characterised in that** the planar non-woven fabric lay (2) has a mass per unit area of between 5 g/m² and 50 g/m², preferably of between 20 g/m² and 40 g/m².

7. The composite web (1) according to one of the Claims 1 to 6, **characterised in that** the laminated strips (4) are bonded with the planar non-woven fabric lay (2) with straight or wavy adhesive strips, which run along the direction of the laminated strips (4).

8. The composite web (1) according to one of the Claims 1 to 7, **characterised in that** the film strips (5) and the non-woven fabric layer (6) of the laminated strips (4) are not joined over the whole surface, but preferably in the form of strips.

9. The composite web (1) according to one of the Claims 1 to 8, **characterised in that** the non-woven fabric layer (6) of the laminated strips (4) is an elastic spun-bonded non-woven fabric based on core-sheath multifilaments.

10. The composite web (1) according to one of the Claims 1 to 9, **characterised in that** the edge regions, in which the non-woven fabric strips (7) overlap the non-woven fabric layer (6) of the laminated strips (4), have a width of 2 to 7 mm, preferably of more than 4 and less than 5 mm.

11. The composite web (1) according to one of the Claims 1 to 10, **characterised in that** the non-woven fabric strips (7) are bonded with the non-woven fabric layer (6) in the edge regions of the laminated strips (4).

12. The composite web (1) according to Claim 11, **characterised in that** in the edge regions, in which the non-woven fabric strips (7) overlap the non-woven fabric layer (6) of the laminated strips (4), the non-woven fabric strips (7) are not bonded with the underlying non-woven fabric layer (6) of the laminated strip (4) up to a maximum width of 5 mm, preferably of 1 to 4 mm, from the edge of the non-woven fabric strip (7).

13. The composite web (1) according to one of the Claims 1 to 10, **characterised in that** the non-woven fabric strips (7) are joined with the non-woven fabric layer (6) by means of ultrasound welding in the edge regions of the laminated strips (4).

14. The composite web (1) according to one of the Claims 1 to 10, **characterised in that** the non-woven fabric strips (7) consist of a spun-bonded non-woven fabric with a mass per unit area of between 10 and 30 g/m², preferably of more than 10 g/m² and less than 20 g/m².

15. A method for the manufacture of a composite web (1) according to one of the Claims 1 to 14,
wherein spaced apart, parallel running laminated strips (4) of in each case a film strip (5) of an elastic film and a laminated-on non-woven fabric layer (6) on its film side are bonded with a planar non-woven fabric lay (2), wherein
non-woven fabric strips (7) are applied onto the planar non-woven fabric lay (2) between the laminated strips (4), and wherein
the non-woven fabric strips (7) overlap the non-woven fabric layer (6) of the laminated strips (4) in edge regions of the laminated strips (4).

16. The method according to Claim 15, **characterised in that** a laminate (10) of an elastic extruded mono-film of a thermoplastic elastomer and a non-woven fabric layer (6) is drawn off from a roller (11) and cut into laminated strips (4).

17. The method according to Claim 15 or 16, **characterised in that** the film strips (5) and the non-woven fabric layer (6) of each laminated strip (4) are joined by adhesive strips that run in the lengthwise direction of the laminated strip (4).

18. The method according to one of the Claims 15 to 17, **characterised in that** in the region in which the laminated strips (4) are laminated on, adhesive (3) is applied in the form of adhesive strips, which run parallel to the laminated strips (4), and between the laminated strips (4) is applied over the whole surface onto the planar non-woven fabric lay (2).

19. The method according to one of the Claims 15 to 18, **characterised in that** in the region between the laminated strips (4) adhesive (3) is applied over the whole surface onto the planar non-woven fabric lay (2).

20. The method according to one of the Claims 15 to 19, **characterised in that** the non-woven fabric strips (7) are applied onto the non-woven fabric lay (2) such that they overlap the non-woven fabric layer (6) of the laminated strips (4) in the edge regions of the laminated strips (4) with a width of between 2 and 7 mm, preferably of more than 4 and less than 5 mm.

21. The method according to one of the Claims 15 to 20, **characterised in that** adhesive (3) is applied in the form of two adhesive strips in each case onto the non-woven fabric strips (7).

22. The method according to one of the Claims 15 to 21, **characterised in that** adhesive (3) is applied onto the non-woven fabric strips (7) with a separation distance of a maximum of 5 mm, preferably of 1 to 4 mm, from the edge of the non-woven fabric strips (7).

23. The method according to one of the Claims 15 to 20, **characterised in that** the non-woven fabric strips (7) are joined with the non-woven fabric layer (6) by means of ultrasound welding, at least in the edge regions of the laminated strips (4).

24. The method according to one of the Claims 15 to 23, **characterised in that** the composite web (1) is overstretched crosswise to the alignment of the laminated strips (4), preferably in a forging roll arrangement (16).

## Revendications

1. Bande de matériau composite (1) comportant des zones élastiques et non élastiques et qui présente sur ses deux surfaces du non-tissé et par endroits des bandes de film intermédiaires (5) en film élastique, une surface se présentant sous forme d'une couche de non-tissé de surface (2) et la couche de non-tissé (2) étant raccordée entre les bandes de film (5) à du non-tissé qui est disposé sur la surface opposée de la bande de matériau composite (1), **caractérisée en ce que** chaque bande de film (5) est reliée respectivement à une couche de non-tissé (6) pour former une bande de laminé (4), que les bandes de laminé (4) sont collées par leur face en film à la couche de non-tissé de surface (2) et que le non-tissé qui est raccordé entre les bandes de film (5) à la couche de non-tissé de surface (2) se présente sous forme de bandes de non-tissé (7) qui s'étendent parallèlement aux bandes de laminé (4) et chevauchent la couche de non-tissé (6) des bandes de laminé (4) dans les zones périphériques des bandes de laminé (4).

2. Bande de matériau composite (1) selon la revendication 1, **caractérisée en ce que** les bandes de non-tissé (7) sont raccordées ou collées par toute leur surface à la couche de non-tissé de surface (2) par soudage aux ultrasons entre les bandes de laminé (4).

3. Bande de matériau composite (1) selon la revendication 1 ou 2, **caractérisée en ce que** les bandes de film (5) consistent en un monofilm d'élastomère thermoplastique.

4. Bande de matériau composite (1) selon la revendication 3, **caractérisée en ce que** les bandes de film (5) consistent en un polymère du groupe des copolymères de styrène-butadiène-styrène (SBS), copolymères blocs de styrène-isoprène-styrène (SIS), copolymères de styrène-éthènebutène-styrène (SEBS), copolymères de polyéthylène élastiques, copolymères de polypropylène élastiques, copolymères de polyuréthane élastiques, copolymères de polyamide élastiques ou un mélange de ces polymères.

5. Bande de matériau composite (1) selon une des revendications 1 à 4, **caractérisée en ce que** les bandes de film (5) ont une épaisseur comprise entre 5 et 150 µm, de préférence de plus de 10 µm et de moins 70 µm.

6. Bande de matériau composite (1) selon une des revendications 1 à 5, **caractérisée en ce que** la couche de non-tissé de surface (2) présente un grammage compris entre 5 g/m² et 50 g/m², de préférence entre 20 g/m² et 40 g/m².

7. Bande de matériau composite (1) selon une des revendications 1 à 6, **caractérisée en ce que** les bandes de laminé (4) sont collées à la couche de non-tissé de surface (2) par des bandes encollées rectilignes ou de forme ondulée qui s'étendent dans le sens des bandes de laminé (4).

8. Bande de matériau composite (1) selon une des revendications 1 à 7, **caractérisée en ce que** les bandes de film (5) et la couche de non-tissé (6) des bandes de laminé (4) sont raccordées de préférence en forme de bande et pas sur toute leur surface.

9. Bande de matériau composite (1) selon une des revendications 1 à 8, **caractérisée en ce que** la couche de non-tissé (6) des bandes de laminé (4) est un non-tissé élastique à base de multifilaments à noyau-enveloppe.

10. Bande de matériau composite (1) selon une des revendications 1 à 9, **caractérisée en ce que** les zones périphériques dans lesquelles les bandes de non-tissé (7) chevauchent la couche de non-tissé (6) des bandes de laminé (4) ont une largeur de 2 à 7 mm, de préférence de plus de 4 et moins de 5 mm.

11. Bande de matériau composite (1) selon une des revendications 1 à 10, **caractérisée en ce que** les bandes de non-tissé (7) sont collées à la couche de non-tissé (6) dans les zones périphériques des bandes de laminé (4).

12. Bande de matériau composite (1) selon la revendication 11, **caractérisée en ce que** les bandes de non-tissé (7), dans les zones périphériques où les bandes de non-tissé (7) chevauchent la couche de non-tissé (6) des bandes de laminé (4), en partant du bord de la bande de non-tissé (7), sur une largeur de 5 mm au maximum, de préférence 1 à 4 mm, ne sont pas collées à la couche de non-tissé sous-jacente (6) de la bande de laminé (4).

13. Bande de matériau composite (1) selon une des revendications 1 à 10, **caractérisée en ce que** les bandes de non-tissé (7), dans les zones périphériques des bandes de laminé (4), sont reliées par soudage aux ultrasons à la couche de non-tissé (6).

14. Bande de matériau composite (1) selon une des revendications 1 à 10, **caractérisée en ce que** les bandes de non-tissé (7) consistent en un non-tissé ayant un grammage compris entre 10 et 30 g/m², de préférence de plus de 10 g/m² et de moins de 20 g/m².

15. Procédé de fabrication d'une bande de matériau composite (1) selon une des revendications 1 à 14,
dans lequel des bandes de laminé (4) espacées et orientées parallèlement faites respectivement d'une bande de film (5) en film élastique et d'une couche plaquée de non-tissé (6) sont collées par leur face en film à une couche de non-tissé de surface (2),
dans lequel des bandes de non-tissé (7) sont appliquées entre les bandes de laminé (4) sur la couche de non-tissé de surface (2) et
dans lequel les bandes de non-tissé (7) chevauchent la couche de non-tissé (6) des bandes de laminé (4) dans les zones périphériques des bandes de laminé (4).

16. Procédé selon la revendication 15, **caractérisé en ce qu'**un laminé (10) en monofilm extrudé élastique en élastomère thermoplastique et en couche de non-tissé (6) est tiré d'un rouleau (11) et découpé en bandes de laminé (4).

17. Procédé selon la revendication 15 ou 16, **caractérisé en ce que** la bande de film (5) et la couche de non-tissé (6) de chaque bande de laminé (4) sont reliées par des bandes encollées qui s'étendent dans le sens longitudinal de la bande de laminé (4).

18. Procédé selon une des revendications 15 à 17, **caractérisé en ce que** de la colle (3) est appliquée dans la zone où les bandes de laminé (4) sont plaquées sous forme de bandes encollées qui s'étendent parallèlement aux bandes de laminé (4) et sur toute la surface de la couche de non-tissé de surface (2) entre les bandes de laminé (4).

19. Procédé selon une des revendications 15 à 18, **caractérisé en ce que** de la colle (3) est appliquée sur toute la surface de la couche de non-tissé de surface (2) dans la zone située entre les bandes de laminé (4).

20. Procédé selon une des revendications 15 à 19, **caractérisé en ce que** les bandes de non-tissé (7) sont appliquées sur la couche de non-tissé (2) de manière à chevaucher la couche de non-tissé (6) des bandes de laminé (4) dans les zones périphériques des bandes de laminé (4) avec une largeur comprise entre 2 et 7 mm, de préférence de plus de 4 et de moins de 5 mm.

21. Procédé selon une des revendications 15 à 20, **caractérisé en ce que** la colle (3) est appliquée sous forme de respectivement deux bandes encollées sur les bandes de non-tissé (7).

22. Procédé selon une des revendications 15 à 21, **caractérisé en ce que** la colle (3) est appliquée sur les bandes de non-tissé (7) à une distance de 5 mm au maximum, de préférence 1 à 4 mm, du bord des bandes de non-tissé (7).

23. Procédé selon une des revendications 15 à 20, **caractérisé en ce que** les bandes de non-tissé (7) sont raccordées à la couche de non-tissé (6) par soudage aux ultrasons au moins dans les zones périphériques des bandes de laminé (4).

24. Procédé selon une des revendications 15 à 23, **caractérisé en ce que** la bande de matériau composite (1) est sur-étirée transversalement à l'orientation des bandes de laminé (4), de préférence dans un dispositif de laminoir à étirer (16).
